(19) Europäisches Patentamt
European Patent Office
Office européen des brevets

(11) EP 1 253 136 A1

(12) DEMANDE DE BREVET EUROPEEN

(43) Date de publication:
**30.10.2002 Bulletin 2002/44**

(51) Int Cl.7: **C07C 213/08**

(21) Numéro de dépôt: **02290903.0**

(22) Date de dépôt: **11.04.2002**

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **27.04.2001 FR 0105701**

(71) Demandeur: **Atofina**
**92800 Puteaux (FR)**

(72) Inventeurs:
- **Druzkowski, Thierry**
  **75011 Paris (FR)**
- **Herbst, Gilles**
  **54000 Nancy (FR)**
- **Tretjak, Serge**
  **57520 Roulhing (FR)**
- **Riondel, Alain**
  **57600 Forbach (FR)**

(74) Mandataire: **Rieux, Michel et al**
**ATOFINA,**
**4/8, cours Michelet,**
**La Défense 10**
**92091 Paris La Défense Cedex (FR)**

# (54) Procédé de fabrication de solutions aqueuses de sels insaturés d'ammonium quaternaire

(57) Ce procédé de fabrication d'une solution aqueuse du sel d'ammonium quaternaire insaturé, répondant à la formule (I),par réaction, en présence d'eau, de l'acrylate de N,N-diméthylaminoéthyle (ADAME) avec un agent quaternisant de formule (II), est caractérisé par le fait que l'on conduit ladite réaction en continu dans une colonne agitée à disques rotatifs, avec introduction de l'agent quaternisant en pied de colonne et introduction de l'ADAME et de l'eau en tête de colonne, ladite réaction étant conduite à une température de 35 à 60°C et sous une pression de 10 à 20 bars.

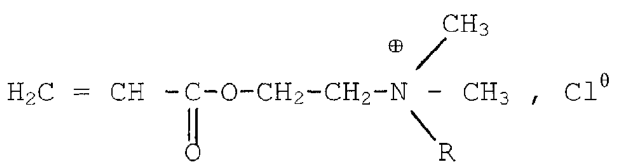

$$R - Cl$$

(I) (II)

R = radical méthyle ou benzyle.

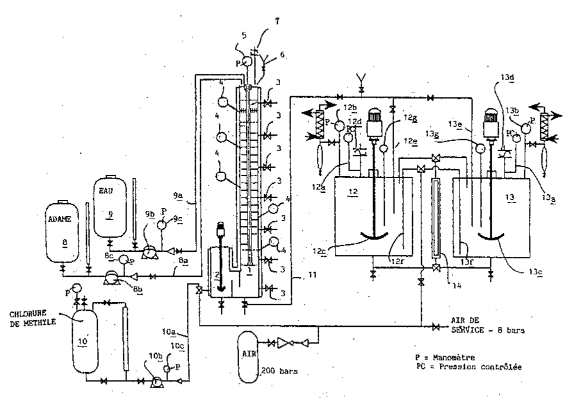




EP 1 253 136 A1

## Description

La présente invention porte sur la fabrication de solutions aqueuses de sels insaturés d'ammonium quaternaire (ci-après dénommés sels quaternaires), répondant à la formule (I) suivante :

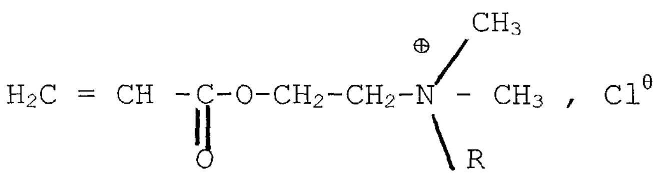

(I)

dans laquelle R représente méthyle ou benzyle,
par réaction, en présence d'eau, de l'acrylate de N,N-diméthylaminoéthyle (ADAME) avec un agent quaternisant de formule (II) :

$$R - Cl \qquad (II)$$

dans laquelle R est tel que défini ci-dessus.

On utilise des solutions aqueuses de sels quaternaires (I) pour préparer des polymères destinés à servir de floculants cationiques dans le traitement des eaux.

Le brevet européen EP-B-250 325 décrit un procédé de préparation de solutions aqueuse de sels quaternaires dont ceux de formule (I), procédé selon lequel, en présence d'au moins un inhibiteur de polymérisation :

- dans une première étape (a), on fait réagir l'ADAME avec 5 à 20% en poids de la quantité pondérale nécessaire à la réaction de l'agent quaternisant, ou, suivant une variante (a'), avec 5 à 20% en poids, par rapport au poids de l'ADAME, d'une solution aqueuse de sels quaternaires, laquelle comprend de 50 à 85% en poids de sels quaternaires ; et
- dans une deuxième étape (b), on ajoute en continu l'eau et l'agent quaternisant jusqu'à l'obtention de la concentration souhaitée de sels quaternaires dans l'eau.

Pendant les étapes (a) et (b), on maintient la température à une valeur comprise entre 30 et 60°C. De plus, pendant les étapes (a) et (b) et en particulier à l'approche de la fin de la réaction, on maintient dans le milieu réactionnel un courant de gaz oxygéné tel que le rapport en volume (ou débit volumétrique) de gaz total à la sortie du réacteur sur le volume (ou débit volumétrique) d'oxygène introduit à l'entrée de ce même réacteur est inférieur à 100.

Ce procédé permet de préparer des solutions aqueuses de sels quaternaires qui ont une stabilité à température ambiante supérieure à un an. Toutefois, on constate, dans ces solutions, une teneur particulièrement élevée d'impuretés, en particulier de

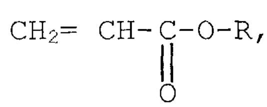

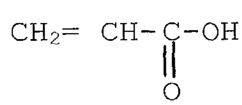

et d'ADAME. En outre, ce procédé nécessite destemps de réaction relativement longs, ce qui représente un inconvénient économique évident.

Dans la demande internationale WO 89/07 588, a alors été proposé un procédé destiné à réduire la formation des impuretés lors de la réaction de quaternisation. Conformément à ce procédé, la réaction est effectuée à une température comprise entre 10 et 80°C, et

- dans une première étape, on introduit dans le réacteur la totalité ou une partie de l'agent quaternisant nécessaire à la réaction, cet agent étant à l'état liquide dans les conditions de la réaction,

- ensuite, on ajoute l'ADAME, et
- dès que 0 à 30% de la stoechiométrie de l'ADAME ont été introduits dans le réacteur, on ajoute en continu et simultanément le reste d'agent quaternisant, le reste d'ADAME et l'eau jusqu'à l'obtention de la concentration souhaitée de sels quaternaires,
- et, dans le cas où l'agent quaternisant est introduit à l'état gazeux à la température de réaction, la réaction est effectuée en présence d'oxygène et on impose une pression de manière que l'agent quaternisant soit liquide à la température de réaction, et, en fin de réaction, on diminue progressivement la pression jusqu'à la pression atmosphérique et simultanément on impose un rapport en débit volumétrique de gaz total à la sortie du réacteur sur le débit volumétrique d'oxygène introduit dans le réacteur inférieur à 100.

Le procédé ci-dessus selon WO 89/07 588 apporte des améliorations notables au procédé selon EP-B-250 325. Cependant, il est apparu que la pureté avec laquelle on obtient les sels quaternaires est encore insuffisante. Ainsi, au cours de la réaction de l'ADAME avec $CH_3Cl$ en milieu aqueux, conduisant au sel désigné également dans ce qui suit par l'abréviation ADAMQUAT MC, il se forme, comme impuretés, outre l'acide acrylique (AA) formé par hydrolyse de l'ADAME, le dimère de l'ADAMQUAT MC, représenté par la formule (1) :

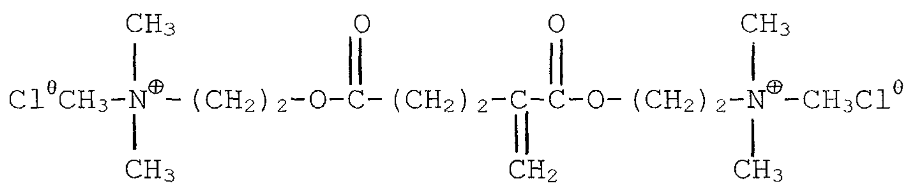
(1).

Grâce à une série de tests de réactivité en polymérisation, il a pu être démontré que ces impuretés affectaient la qualité des polymères cationiques dérivés de l'ADAMQUAT.

La Société déposante a donc recherché des conditions opératoires de préparation de solutions aqueuses du sel de formule (I), qui soient capables de minimiser des impuretés précitées, de façon à proposer un sel (I) en solution aqueuse de très haute qualité analytique.

Ce nouveau procédé, qui fait donc l'objet de la présente invention et qui présente l'avantage complémentaire important de fournir un produit de qualité constante, est caractérisé par le fait que l'on conduit ladite réaction en continu dans une colonne agitée à disques rotatifs, avec introduction de l'agent quaternisant en pied de colonne et introduction de l'ADAME et de l'eau en tête de colonne, ladite réaction étant conduite à une température de 35 à 60°C, de préférence à une température de 40 à 50°C, et sous une pression de 10 à 20 bars, de préférence de 12 à 16 bars.

Par ailleurs, on conduit généralement la réaction avec un rapport molaire de $CH_3Cl$ à l'ADAME qui est compris entre 1 et 1,5, en particulier entre 1,02 et 1,15, et un temps de séjour de 2 à 6 heures, en particulier de 2,5 à 3,5 heures. Quant au rapport des débits d'introduction eau/agent quaternisant, il est généralement compris entre 0,3 et 1,2, en particulier entre 0,5 et 0,9.

Le procédé selon la présente invention est par ailleurs avantageusement conduit en présence d'au moins un stabilisant, choisi notamment parmi le 3,5-ditert.-butyl-4-hydroxytoluène, l'éther méthylique d'hydroquinone, l'hydroquinone, le catéchol, le tert.-butyl catéchol et les mélanges de ces stabilisants, la teneur en agent(s) stabilisant(s) étant notamment de 20 à 2000 ppm, de préférence de 100 à 1200 ppm par rapport à la solution aqueuse de sel quaternaire (I).

Le procédé selon l'invention est en outre avantageusement conduit en présence d'au moins un agent séquestrant pour métaux, choisi notamment parmi l'acide diéthylène triamine penta acétique, le sel pentasodique de l'acide diéthylène triamine penta acétique, l'acide N-hydroxyéthyl-éthylène diamine triacétique et le sel trisodique de l'acide N-hydroxyéthyl-éthylène diamine triacétique, la teneur en agent(s) séquestrant(s) étant notamment de 1 à 100 ppm, de préférence de 5 à 30 ppm, par rapport à la solution aqueuse de sel quaternaire (I).

D'une manière générale, les agents séquestrants sont ajoutés sous la forme d'une solution aqueuse, car ils sont généralement disponibles sous cette forme. Ainsi, le sel pentasodique de l'acide diéthylène triamine penta acétique commercialisé sous la dénomination VERSENEX 80 se présente sous la forme d'une solution aqueuse à 40% en poids environ.

Le procédé selon l'invention permet notamment de préparer des solutions aqueuses ayant une concentration en sels quaternaires (I) de 50 à 85% en poids, et contenant des quantités très faibles d'impuretés, comme illustré dans le Tableau 1 ci-après.

Les Exemples suivants illustrent la présente invention sans toutefois en limiter la portée. Dans ces exemples, les pourcentages sont en poids sauf indication contraire.

La Figure unique du dessin annexé représente l'installation utilisée pour la fabrication en continu des solutions aqueuses de chlorure d'acryloyloxytriméthyl-ammonium selon les Exemples. Cette installation est décrite à l'Exemple

1.

EXEMPLE 1 : Fabrication en continu d'une solution aqueuse d'ADAMQUAT MC

On utilise une colonne agitée à disques rotatifs (1) constituée d'un tube en acier inoxydable d'une capacité de 0,5 litre, comportant une zone (2) de prémélangeage des réactifs (1/10 du volume total) et équipé de sept prises d'échantillons (3), de cinq prises de température (4), d'un manomètre (5), d'une soupape d'équerre tarée à 17 bars (6), d'un disque d'éclatement taré à 25 bars (7), et de piquages permettant l'introduction des réactifs.

Chacun des trois bacs d'alimentation (8 ; 9 ; 10) respectivement en ADAME, eau et chlorure de méthyle est relié à la colonne agitée (1) par une conduite d'alimentation (respectivement 8a ; 9a ; 10a) avec interposition d'une pompe d'alimentation (8b ; 9b ; 10b) et d'un manomètre (respectivement 8c ; 9c; 10c). La conduite (10a) d'alimentation en chlorure de méthyle débouche en pied de colonne, et les deux conduites d'alimentation en ADAME et eau (respectivement (8a ; 9a) débouchent en tête de colonne.

En fonctionnement, l'ADAMQUAT MC en solution aqueuse, dont la densité est supérieure à celle de l'ADAME et à celle du chlorure de méthyle, sort par le bas de la colonne (1) et est déversé par une conduite de sortie (11) dans l'une des deux capacités (12 ; 13) équipées chacune d'un régulateur de pression (12a ; 13a), d'un manomètre (12b ; 13b), d'un agitateur spécifique (12c ; 13c) fonctionnant sous pression, d'une soupape d'éclatement tarée à 25 bars (12d ; 13d), d'un dégazeur (12e, 13e), d'une introduction d'air (12f ; 13f), d'un niveau (14) et de prises de température (12g, 13g). La capacité dans laquelle est dirigé l'ADAMQUAT MC en solution aqueuse est utilisée comme dégazeur. Une fois remplie, elle est isolée et son contenu est dégazé puis déchargé, l'ADAMQUAT MC en solution aqueuse sortant de la colonne (1) étant alors dirigé sur l'autre capacité où il sera dégazé puis déchargé. Autrement dit, la colonne fonctionne en continu et, pour l'opération de dégazage, le montage fonctionne en semi-continu.

Le mode opératoire est le suivant :

550 g d'ADAMQUAT MC 80 fini (solution aqueuse à 80%) sont chargés dans la colonne (1). Le montage est mis sous pression (11 bars) à l'aide d'une bouteille d'air appauvri à 200 bars. Une fois la colonne (1) mise à la température de travail souhaitée de 45°C, on démarre les trois pompes d'alimentation (8b ; 9b ; 10b) et on règle leur débit en fonction du temps de séjour moyen de 2,93 heures et du rapport molaire $CH_3Cl$/ADAME visé de 1,18. A cet effet, le débit d'introduction de l'ADAME est de 120 ml/h, celui de l'eau est de 35 ml/h, et celui du chlorure de méthyle, entre 49 et 53 ml/h. Dans ces conditions, le rapport molaire $CH_3Cl$/ADAME se situe entre 1,14 et 1,23 (rapport molaire moyen : 1,18), et le rapport des débits d'alimentation Eau/$CH_3Cl$, entre 0,71 et 0,78, et le temps de séjour est de 2,93 heures.

Les résultats sont donnés dans le Tableau 1 suivant. Le taux de conversion de l'ADAME est resté à 100% et le régime d'équilibre est atteint après 7,5 heures de fonctionnement.

Les techniques d'analyse étaient les suivantes :

- ☐ Le pourcentage d'ADAMQUAT MC a été déterminé par potentiométrie, permettant de doser les ions chlorure au moyen de nitrate d'argent.
- ☐ Les impuretés AA et dimère de l'ADAMQUAT MC ont été analysées par Chromatographie Liquide Haute Performance (HPLC).

EXEMPLE 2

On a procédé comme à l'Exemple 1, excepté que l'on a diminué le rapport molaire $CH_3Cl$/ADAME. Les conditions opératoires et les résultats sont également rapportés dans le Tableau 1. Le taux de conversion de l'ADAME est resté à 100% pour une durée de mise en régime de 6 heures.

Tableau 1

| Exemple | Tempé-rature (°C) | Pression (bars) | Débit des réactifs (ml/h) | | | Temps de séjour (h) | Rapport molaire moyen $CH_3Cl$/ADAME | Rapport des débits d'alimentation Eau/$CH_3Cl$ | Résultats | | | | |
|---|---|---|---|---|---|---|---|---|---|---|---|---|---|
| | | | ADAME | Eau | $CH_3Cl$ | | | | Durée (min.) | ADAMQUAT MC (%) | Eau (%) | AA* (ppm) | Dimère de l'ADAMQUAT MC** (ppm) |
| 1 | 45 | 11 | 120 | 35 | 49-53 | 2,93 | 1,18 | 0,71-0,78 | charge | 78,4 | 20,4 | 2001 | 1456 |
| | | | | | | | | | 105 | 81 | 27,1 | 1552 | 1515 |
| | | | | | | | | | 195 | 72,8 | 27,1 | 820 | 328 |
| | | | | | | | | | 315 | 76,3 | 22,5 | 531 | 141 |
| | | | | | | | | | 450 | 80,5 | 18,2 | 329 | / |
| | | | | | | | | | 585 | 82,9 | 17,6 | 237 | / |
| | | | | | | | | | 635 | 82,8 | 20,4 | 288 | / |
| 2 | 45 | 11 | 120 | 35 | 44-47 | 3 | 1,05 | 0,81-0,86 | charge | 81,3 | 19,7 | 1221 | 1309 |
| | | | | | | | | | 120 | 74,2 | 26,2 | 1100 | 1181 |
| | | | | | | | | | 180 | 77 | 23,9 | 669 | 408 |
| | | | | | | | | | 370 | 80,4 | 20,3 | 360 | 149 |
| | | | | | | | | | 555 | 80,4 | 19,8 | 214 | / |

* AA = Acide Acrylique ; ** ADAMQUAT MC = chlorure d'acryloyloxytriméthylammonium

**Revendications**

1. Procédé de fabrication d'une solution aqueuse du sel d'ammonium quaternaire insaturé, répondant à la formule (I) suivante :

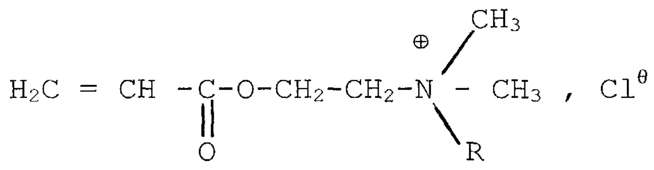

(I)

dans laquelle R représente un radical méthyle ou benzyle,par réaction, en présence d'eau, de l'acrylate de N,N-diméthylaminoéthyle (ADAME) avec un agent quaternisant de formule (II) :

$$R - Cl \qquad (II)$$

dans laquelle R est tel que défini ci-dessus,
**caractérisé par le fait que** l'on conduit ladite réaction en continu dans une colonne agitée à disques rotatifs, avec introduction de l'agent quaternisant en pied de colonne et introduction de l'ADAME et de l'eau en tête de colonne, ladite réaction étant conduite à une température de 35 à 60°C et sous une pression de 10 à 20 bars.

2. Procédé selon la revendication 1, **caractérisé par le fait que** l'on conduit la réaction à une température de 40 à 50°C.

3. Procédé selon l'une des revendications 1 et 2, **caractérisé par le fait que** l'on conduit la réaction sous une pression de 12 à 16 bars.

4. Procédé selon l'une des revendications 1 à 3, **caractérisé par le fait que** l'on conduit la réaction avec un rapport molaire de $CH_3Cl$ à l'ADAME qui est compris entre 1 et 1,5.

5. Procédé selon la revendication 4, **caractérisé par le fait que** l'on conduit la réaction avec un rapport molaire de l'agent quaternisant à l'ADAME qui est compris entre 1,02 et 1,15.

6. Procédé selon l'une des revendications 1 à 5, **caractérisé par le fait que** l'on conduit la réaction avec un rapport des débits d'introduction eau/agent quaternisant compris entre 0,3 et 1,2, en particulier entre 0,5 et 0,9.

7. Procédé selon l'une des revendications 1 à 6, **caractérisé par le fait que** l'on conduit la réaction avec un temps de séjour de 2 à 6 heures.

8. Procédé selon la revendication 7, **caractérisé par le fait que** l'on conduit la réaction avec un temps de séjour de 2,5 à 3,5 heures.

9. Procédé selon l'une des revendications 1 à 8, **caractérisé par le fait qu'**il est conduit en présence d'au moins un stabilisant, choisi notamment parmi le 3,5-ditert.-butyl-4-hydroxytoluène, l'éther méthylique d'hydroquinone, l'hydroquinone, le catéchol, le tert.-butyl catéchol et les mélanges de ces stabilisants, la teneur en agent(s) stabilisant (s) étant notamment de 20 à 200 ppm, de préférence de 100 à 1200 ppm par rapport à la solution aqueuse de sel quaternaire (I).

10. Procédé selon la revendication 9, **caractérisé par le fait qu'**il est conduit en outre en présence d'au moins un agent séquestrant pour métaux, choisi notamment parmi l'acide diéthylène triamine penta acétique, le sel pentasodique de l'acide diéthylène triamine penta acétique, l'acide N-hydroxyéthyl-éthylène diamine triacétique et le sel trisodique de l'acide N-hydroxyéthyl-éthylène diamine triacétique, la teneur en agent(s) séquestrant(s) étant notamment de 1 à 100 ppm, de préférence de 5 à 30 ppm, par rapport à la solution aqueuse de sel quaternaire (I).

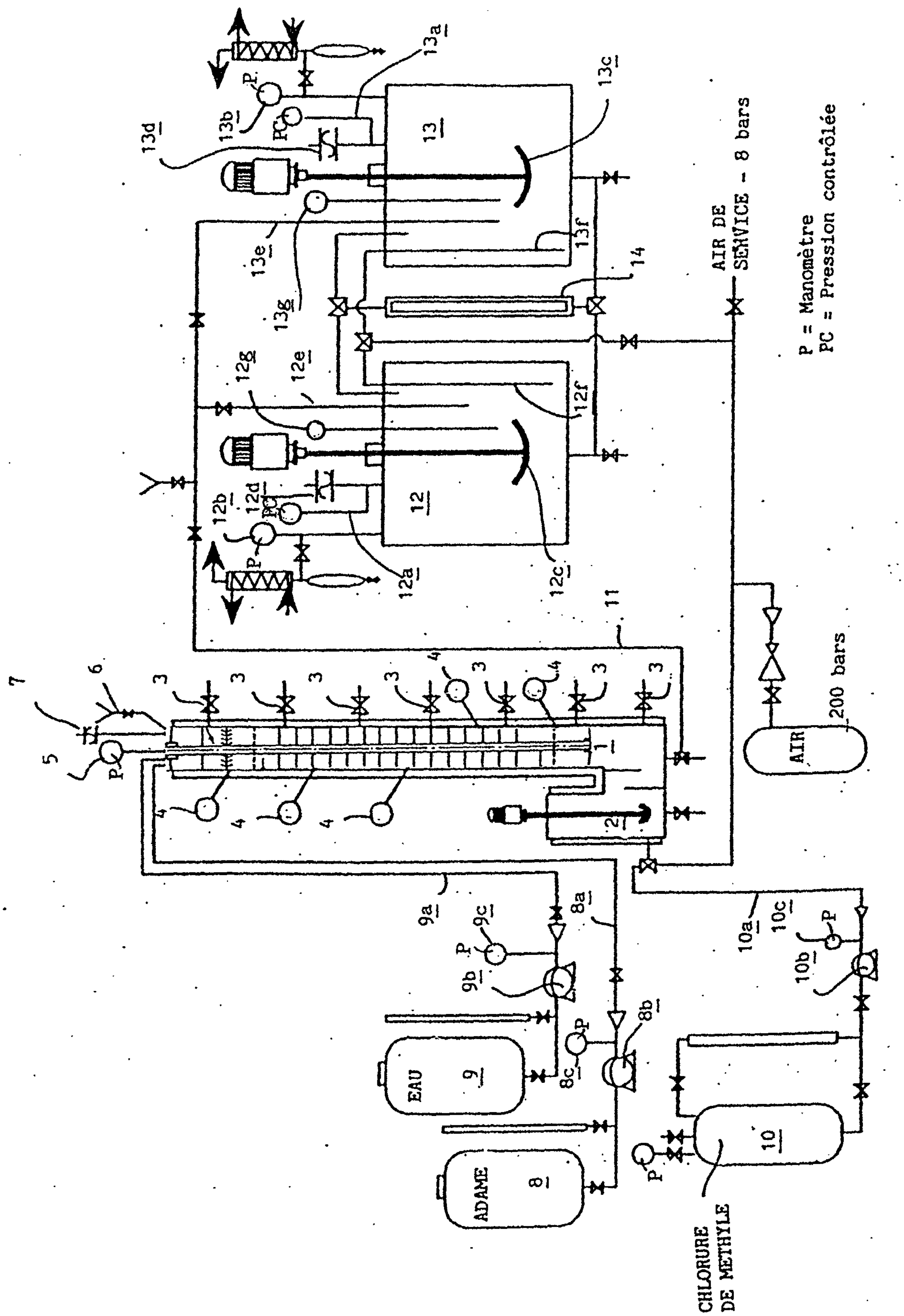
ADAME
8
EAU
9
CHLORURE
DE METHYLE
10
AIR
200 bars
AIR DE
SERVICE - 8 bars
P = Manomètre
PC = Pression contrôlée
1
2
3
4
5
6
7
8a
8b
8c
9a
9b
9c
10a
10b
10c
11
12
12a
12b
12c
12d
12e
12f
12g
13
13a
13b
13c
13d
13e
13f
13g
14
P
PC

Office européen des brevets

# RAPPORT DE RECHERCHE EUROPEENNE

Numéro de la demande
EP 02 29 0903

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | FR 2 788 767 A (ATOCHEM ELF SA) 28 juillet 2000 (2000-07-28) * page 3, ligne 30 - page 4, ligne 14 * * tableau 1 * * exemple 1 * | 1-10 | C07C213/08 |
| D,A | WO 89 07588 A (NORSOLOR SA) 24 août 1989 (1989-08-24) * page 2, ligne 11 - page 3, ligne 9 * | 1-10 | |
| D,A | EP 0 250 325 A (CHARBONNAGES STE CHIMIQUE) 23 décembre 1987 (1987-12-23) * page 2, ligne 57 - page 3, ligne 20 * | 1-10 | |
| A | US 5 912 383 A (LEGROS ROBERT ET AL) 15 juin 1999 (1999-06-15) * colonne 2, ligne 15 - ligne 37 * | 1-10 | |

DOMAINES TECHNIQUES RECHERCHES (Int.Cl.7)
C07C

Le présent rapport a été établi pour toutes les revendications

| Lieu de la recherche | Date d'achèvement de la recherche | Examinateur |
|---|---|---|
| LA HAYE | 20 août 2002 | O'Sullivan, P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 02 29 0903

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

20-08-2002

| Document brevet cité au rapport de recherche | Date de publication | Membre(s) de la famille de brevet(s) | Date de publication |
|---|---|---|---|
| FR 2788767 A | 28-07-2000 | FR 2788767 A1 | 28-07-2000 |
| | | AU 3056900 A | 07-08-2000 |
| | | EP 1104400 A1 | 06-06-2001 |
| | | WO 0043348 A1 | 27-07-2000 |
| WO 8907588 A | 24-08-1989 | FR 2627181 A1 | 18-08-1989 |
| | | AT 75724 T | 15-05-1992 |
| | | CA 1333614 A1 | 20-12-1994 |
| | | DE 68901398 D1 | 11-06-1992 |
| | | EP 0329512 A1 | 23-08-1989 |
| | | ES 2031367 T3 | 01-12-1992 |
| | | WO 8907588 A1 | 24-08-1989 |
| | | GR 3005346 T3 | 24-05-1993 |
| | | JP 2776597 B2 | 16-07-1998 |
| | | JP 3502448 T | 06-06-1991 |
| | | US 5260480 A | 09-11-1993 |
| EP 0250325 A | 23-12-1987 | US 4745214 A | 17-05-1988 |
| | | AT 72228 T | 15-02-1992 |
| | | DE 3776423 D1 | 12-03-1992 |
| | | EP 0250325 A2 | 23-12-1987 |
| | | ES 2033333 T3 | 16-03-1993 |
| | | GR 3004454 T3 | 31-03-1993 |
| | | JP 2547381 B2 | 23-10-1996 |
| | | JP 63005064 A | 11-01-1988 |
| US 5912383 A | 15-06-1999 | FR 2750696 A1 | 09-01-1998 |
| | | AT 207875 T | 15-11-2001 |
| | | AU 2847497 A | 15-01-1998 |
| | | CA 2209769 A1 | 08-01-1998 |
| | | CN 1174188 A | 25-02-1998 |
| | | CZ 9702140 A3 | 13-05-1998 |
| | | DE 69707769 D1 | 06-12-2001 |
| | | DE 69707769 T2 | 11-07-2002 |
| | | EP 0819671 A1 | 21-01-1998 |
| | | JP 2945356 B2 | 06-09-1999 |
| | | JP 10067721 A | 10-03-1998 |

EPO FORM P0460

Pour tout renseignement concernant cette annexe : voir Journal Officiel de l'Office européen des brevets, No.12/82